# EUROPEAN PATENT SPECIFICATION

(11) **EP 1 409 013 B1**
(45) Date of publication and mention of the grant of the patent: **18.11.2009**
(21) Application number: 02767756.6
(22) Date of filing: 26.07.2002
(51) Int. Cl.: A61K 39/39, A61P 31/00

(54) **VACCINES COMPRISING ALUMINIUM ADJUVANTS AND HISTIDINE**
ALUMINIUMADJUVANS UND HISTIDIN ENTHALTENDE IMPSTOFFE
VACCINS COMPRENANT DES ADJUVANTS ALUMINIUM ET HISTIDINE

(30) Priority: 26.07.2001 GB 0118249; 20.06.2002 WO PCT/IB02/03191
(43) Date of publication of application: 21.04.2004
(73) Proprietor: Novartis Vaccines and Diagnostics S.r.l., 53100 Siena (SI) (IT)
(72) Inventor: CONTORNI, Mario, 53100 Siena (IT); MAFFEI, Massimo, 53100 Siena (IT)
(74) Representative: Marshall, Cameron John
(86) International application number: PCT/IB2002/003495
(87) International publication number: WO 2003/009869

(56) References cited:
- EP-A- 0 835 663
- WO-A-00/57906
- WO-A-01/41800
- WO-A-99/48525
- HO M M ET AL: "Assessment of the stability and immunogenicity of meningococcal oligosaccharide C-CRM197 conjugate vaccines" VACCINE, BUTTERWORTH SCIENTIFIC. GUILDFORD, GB, vol. 19, no. 7-8, 22 November 2000 (2000-11-22), pages 716-725, XP004225388 ISSN: 0264-410X

## Description

### TECHNICAL FIELD

This invention is in the field of vaccine formulation.

### BACKGROUND ART

As well as containing antigenic substances, vaccines contain substances such as diluents, excipients, preservatives, stabilisers and buffers. Typically, vaccines also contain adjuvants *i*.*e*. a substance which improves the immune response raised in response to the vaccine antigen.

The adjuvants traditionally used in human vaccines have been aluminium salts such as aluminium hydroxide and aluminium phosphate. Many other experimental adjuvants are known and these are reviewed in, for instance, reference 1. Adsorption to aluminium salts remains, however, the most common vaccine adjuvant formulation.

Although their use is widespread, aluminium salts may not always be compatible with particular antigens. It has been suggested, for instance, that aluminium hydroxide may not be suitable for use in multivalent vaccines including hepatitis B virus surface antigen [2] or for use with the capsular polysaccharide from *Haemophilus influenzae* [3]. It has also been suggested that different antigens within the same vaccine formulation should be adsorbed to different aluminium salts [4] for compatibility reasons.

As well as antigen compatibility, it is necessary to consider vaccine stability when using aluminium salts. For instance, their capacity for protein adsorption has been shown to drop over time at room temperature [5] and in response to autoclaving [6]. Alum salts may also cause difficulties in freeze drying [7]. Furthermore, it has been found that aluminium hydroxide can hydrolyse saccharide antigens [8], even at low temperatures and when the antigen is conjugated to a carrier protein, thus leading to reduced efficacy.

In general, these issues only arise when attention moves to formulating an antigen for clinical use and may not be appreciated during initial research and development of the antigen itself.

It is an object of the invention to provide improvements in the stability of vaccines which include aluminium salts and, in particular, improvements in pH stability (buffering) and adjuvant adsorption at various temperatures and/or improvements in antigen stability (*e*.*g*. reduction in hydrolysis).

### DISCLOSURE OF THE INVENTION

The invention is defined in the claims and is based on the surprising discovery that the amino acid histidine enhances the stability of vaccines which include aluminium salt adjuvants. This has been found both for adsorbed saccharide antigens and for adsorbed protein antigens.

Histidine has previously been included in aluminium-adjuvanted vaccines. Reference 4 describes the use of L-histidine in Hib/DTPa combination vaccines having antigens adsorbed to an aluminium phosphate adjuvant. Reference 95 describes vaccines in which human papillomavirus vaccine antigens are adsorbed to an aluminium salt and then mixed with a formulation buffer comprising a salt, a histidine buffer and a non-ionic surfactant.

### The antigen

The antigen is preferably a protein antigen or a saccharide antigen (optionally conjugated). Preferred antigens are from bacteria, with the bacterial genus *Neisseria* (*e*.*g*. *N.meningitidis*) being particularly preferred.

Specific bacterial antigens for use with the invention include:
- a protein antigen from *N.meningitidis* serogroup B, such as those in refs. 9 to 15, with protein '287' (see below) and derivatives (*e.g.* 'ΔG287') being particularly preferred,
- an outer-membrane vesicle (OMV) preparation from *N.meningitidis* serogroup B, such as those disclosed in refs. 16, 17, 18, 19 *etc.*
- a saccharide antigen from *N.meningitidis* serogroup A, C, W135 and/or Y, such as the oligosaccharide disclosed in ref. 20 from serogroup C [see also ref. 21].

Further antigens which can be used include:
- a saccharide antigen from *Streptococcus pneumoniae* [*e*.*g*. 22, 23, 24].
- an antigen from *Bordetella pertussis*, such as pertussis holotoxin (PT) and filamentous haemagglutinin (FHA) from *B.pertussis*, optionally also in combination with pertactin and/or agglutinogens 2 and 3 [*e*.*g*. refs. 25 & 26].
- a diphtheria antigen, such as a diphtheria toxoid [*e*.*g*. chapter 3 of ref 27] *e.g.* the CRM₁₉₇ mutant [*e*.*g*. 28].
- a tetanus antigen, such as a tetanus toxoid [*e*.*g*. chapter 4 of ref. 27].
- a protein antigen from *Helicobacter pylori* such as CagA [*e.g.* 29], VacA [*e*.*g*. 29], NAP [*e*.*g*. 30], HopX [*e*.*g*. 31], HopY [*e*.*g*. 31] and/or urease.
- a saccharide antigen from *Haemophilus influenzae B* [*e*.*g*. 21], preferably oligosaccharide.
- an antigen from *N.gonorrhoeae* [*e*.*g.* 9*,* 10, 11].
- an antigen from *Chlamydia pneumoniae* [*e*.*g*. 32, 33, 34, 35, 36, 37, 38].
- an antigen from *Chlamydia trachomatis* [*e.g*. 39].
- an antigen from *Porphyromonas gingivalis* [*e*.*g*. 40].
- an antigen from *Moraxella catarrhalis* [*e*.*g*. 41].
- an antigen from *Streptococcus agalactiae* (group B streptococcus) [*e*.*g*. 42, 43].
- an antigen from *Streptococcus pyogenes* (group A streptococcus) [*e*.*g*. 43, 44, 45].
- an antigen from *Staphylococcus aureus* [*e*.*g*. 46].
- an antigen from *Bacillus anthracis* [*e*.*g*. 47, 48, 49].
- an antigen from hepatitis A virus, such as inactivated virus [*e*.*g*. 50, 51].
- an antigen from hepatitis B virus, such as the surface and/or core antigens [*e*.*g*. 51,52].
- an antigen from hepatitis C virus [*e*.*g*. 53].
- polio antigen(s) [*e.g.* 54, 55] such as IPV.
- rabies antigen(s) [*e*.*g*. 56] such as lyophilised inactivated virus [e.g.57, RabAvert™].
- measles, mumps and/or rubella antigens [*e*.*g*. chapters 9, 10 & 11 of ref. 27].
- influenza antigen(s) [*e*.*g*. chapter 19 of ref. 27], such as the haemagglutinin and/or neuraminidase surface proteins.
- an antigen from a virus in the flaviviridae family (genus flavivirus), such as from yellow fever virus, Japanese encephalitis virus, four serotypes of Dengue viruses, tick-borne encephalitis virus, West Nile virus.
- a pestivirus antigen, such as from classical porcine fever virus, bovine viral diarrhoea virus, and/or border disease virus.
- a parvovirus antigen *e*.*g*. from parvovirus B 19.

The composition may comprise one or more of these further bacterial and viral antigens. The composition may comprise no viral antigens.

Other antigens which may be used include:
- a prion protein (*e*.*g*. the CJD prion protein)
- an amyloid protein, such as a beta peptide [58]
- a cancer antigen, such as those listed in Table 1 of ref. 59 or in tables 3 & 4 of ref. 60.

Where a saccharide or carbohydrate antigen is used, it is preferably conjugated to a carrier protein in order to enhance immunogenicity [*e*.*g*. refs. 61 to 70]. Preferred carrier proteins are bacterial toxins or toxoids, such as diphtheria or tetanus toxoids. The CRM₁₉₇ diphtheria toxoid is particularly preferred. Other suitable carrier proteins include the *N.meningitidis* outer membrane protein [*e*.*g*. ref. 71], synthetic peptides [*e*.*g*. 72, 73], heat shock proteins *[e.g.* 74], pertussis proteins [*e*.*g*. 75, 76], protein D from *H.influenzae* [*e*.*g*. 77], toxin A or B from *C.difficile* [*e*.*g*. 78], *etc.* Where a mixture comprises capsular saccharides from both serogroups A and C, it is preferred that the ratio (w/w) of MenA saccharide:MenC saccharide is greater than 1 (*e*.*g*. 2:1, 3:1, 4:1, 5:1, 10:1 or higher). Saccharides from different serogroups of *N.meningitidis* may be conjugated to the same or different carrier proteins.

Any suitable conjugation reaction can be used, with any suitable linker where necessary.

Toxic protein antigens may be detoxified where necessary (*e*.*g*. detoxification of pertussis toxin by chemical and/or genetic means [26]).

Human papilloma virus (HPV) virus-like particles (VLPs) are not preferred antigens (*cf.* WO00/45841, WO00/57906, WO01/28585).

Where a diphtheria antigen is included in the composition it is preferred also to include tetanus antigen and pertussis antigens. Similarly, where a tetanus antigen is included it is preferred also to include diphtheria and pertussis antigens. Similarly, where a pertussis antigen is included it is preferred also to include diphtheria and tetanus antigens. Whole cell pertussis antigen may be used.

Where HBsAg is present, preferably it is either adsorbed to aluminium hydroxyphosphate or is not adsorbed to any salt. Adsorption of HBsAg to an aluminium hydroxide is preferably avoided.

Where a *H.influenzae* saccharide antigen is present, preferably it is either adsorbed to aluminium hydroxyphosphate or is not adsorbed to any salt. Adsorption of Hib saccharides to an aluminium hydroxide is preferably avoided.

Antigens in the composition will typically be present at a concentration of at least 1µg/ml each. In general, the concentration of any given antigen will be sufficient to elicit an immune response against that antigen.

As an alternative to using proteins antigens in the composition of the invention, nucleic acid encoding the antigen may be used [*e*.*g*. refs. 79 to 87]. Protein components of the compositions of the invention may thus be replaced by nucleic acid (preferably DNA *e.g.* in the form of a plasmid) that encodes the protein.

### The aluminium salt

The aluminium salt is preferably an aluminium hydroxide (*e*.*g*. aluminium oxyhydroxide) or an aluminium phosphate (*e.g*. aluminium hydroxyphosphate or orthophosphate), but any other suitable salt may also be used (*e*.*g*. sulphate *etc*. [*e*.*g*. see chapters 8 & 9 of ref. 1]). The salt may take any suitable form (*e*.*g*. gel, crystalline, amorphous *etc*.)*.* Preferred salts are (amorphous) hydroxyphosphates and (crystalline) oxyhydroxide (boehmite).

Hydroxyphosphates are obtained by precipitation and the reaction conditions and reactant concentrations during the precipitation reaction influence the degree of substitution of phosphate for hydroxyl in the salt. Hydroxyphosphates generally have a PO₄/Al molar ratio between 0.3 and 0.99, and preferred salts have a ratio between 0.8 and 0.95 (*e*.*g*. 0.88±0.05). Hydroxyphosphates [Al(OH)ₓ(PO₄)_{y}, wherein the sum of the valence of each anion times its mole fraction is -3] can be distinguished from AlPO₄ by the presence of hydroxyl groups. For example, an IR spectrum band at 3146cm⁻¹ (*e*.*g*. when heated to 200°C) indicates the presence of structural hydroxyls.

Aluminium oxyhydroxide [AlO(OH)] can be distinguished from Al(OH)₃ by IR spectroscopy, in particular by the presence of an adsorption band at 1070cm⁻¹ and a strong shoulder at 3090-3100cm⁻¹,

Mixtures of different aluminium salts may also be used. It is preferred, however, to use essentially a single salt *e*.*g*. where two salts are used, the ratio of one to the other is at least 5:1 by weight *e*.*g*. at least 10:1, 100:1, 1000:1 *etc.*

The salt will generally be present such that the concentration of Al³⁺ is at least 1µg/ml (*e*.*g*. at least 10µg/ml, at least 100µg/ml *etc*.).

The use of histidine in combination with an aluminium phosphate (particularly a hydroxyphosphate) is particularly advantageous for acidic antigens.

### The histidine

Histidine is a standard amino acid and is readily available for use with the invention. As it is inherently biocompatible, it is safe, and thus advantageous as an component in vaccines.

The concentration of histidine in the composition will typically be at least 1µm and at most 1M. The concentration is preferably at least 1mM (*e*.*g*. at least 2mM, 3mM, 4mM, 5mM *etc*.) and is preferably at most 250mM (*e*.*g*. at most 200mM, 150mM, 100mM, 90mM, 80mM, 70mM, 60mM, 50mM, 40mM, 30mM, 20mM, 10mM *etc*.). More preferably the concentration of histidine in the composition is between 2mM and 10mM (*e.g*. between 5mM and 8mM) and, most preferably, it is about 5mM.

The histidine is preferably L-histidine.

The histidine preferably acts as a buffer. Histidine buffers are well known to the skilled person. Accordingly, the histidine may be ionised within the composition of the invention.

The composition preferably has enhanced pH stability and/or reduced antigen hydrolysis when compared to an equivalent composition in which histidine buffer system is either replaced with a sodium phosphate buffer system or in which no buffer system is included. Reduced hydrolysis may be a consequence of enhanced pH stability.

Histidine may be added to the composition in the form of the amino acid itself or in the form of a salt. A typical histidine salt is the monohydrochloride monohydrate.

It will be appreciated that references to histidine in the compositions of the invention refers to 'free' histidine rather than to any histidine residues which may be part of a polypeptide (*e*.*g*. the antigen) within the composition.

### Further characteristics of the composition

The composition is preferably in liquid form, but it may be lyophilised (*cf*. WO01/41800).

The composition may also comprise a sodium salt *e*.*g*. sodium phosphate or sodium chloride. The concentration of the sodium salt is preferably at least 1mM (e.g. at least 2mM, 3mM, 4mM, 5mM etc.) and is preferably at most 10mM (e.g. at most 10mM, 9mM, 8mM, 7mM etc.). More preferably the concentration of sodium salt in the composition is between 1mM and 5mM (e.g. between 2mM and 3mM) and, most preferably, it is about 2.5mM.

A particular advantage of the invention is that it allows good control of pH and adsorption in vaccines which contain high concentrations of free phosphate ions, which ions may be unavoidable in the vaccine *e*.*g*. due to exchange with phosphates in the adjuvant, or due to residual phosphate buffer. Where residual phosphate ions are present at between 3 and 5 mM, for example, pH is difficult to control between 6.0 and 7.0, and some antigens tend to desorb from adjuvants, but the addition of 5 to 10 mM histidine allows pH and adsorption to be controlled, including during storage at elevated temperatures.

The molar ratio of histidine to free phosphate is preferably at least 1.25:1 *e*.*g*. 1.5:1,. 1.75:1, 2:1, 2.25:1, 2.5:1, 3:1, 4:1 *etc.*

The pH of the composition is preferably between 6 and 7 (*e*.*g*. betweem 6.3 and 7.0). The pH may be maintained by the use of a buffer. This will typically be achieved inherently by the histidine in the composition.

The composition will not, in general, contain: serum (*e*.*g*. fetal calf serum *etc*.) or other such components used in cell culture; host cell DNA at a level of greater than 100pg/dose for antigens purified from cell culture; living cells.

The composition will generally be sterile and/or pyrogen-free.

The composition may comprise a detergent (*e*.*g*. a Tween, such as Tween 80) in order to minimise adsorption of antigens to containers.

The composition preferably does not comprise a preservative. Where a preservative is present, mercurial preservatives (*e*.*g*. thimerosal) may be used (*cf*. WO98/34594). Preservatives which may be present or absent are 2-phenoxy-ethanol, methyl parabens, propyl parabens and benzyl alcohol (or mixtures thereof).

### Immunogenic compositions and medicaments

The composition of the invention is typically a vaccine composition.

The invention also provides a composition of the invention for use as a medicament. The medicament is preferably able to raise an immune response in a mammal against the antigen (*i.e.* it is an immunogenic composition) and is more preferably a vaccine.

The invention also provides the use of a composition of the invention in the manufacture of a medicament for raising an immune response in a mammal against the antigen. The medicament is preferably a vaccine.

The immune response is preferably protective. The medicament may raise a booster response.

The mammal is preferably a human, and most preferably a child.

These uses are preferably for the prevention and/or treatment of a disease caused by a *Neisseria* (*e*.*g*. meningitis, septicaemia, gonorrhoea *etc*.), by *H.influenzae* (*e*.*g*. otitis media, bronchitis, pneumonia, cellulitis, pericarditis, meningitis *etc*.) or by pneumococcus (*e*.*g*. meningitis, sepsis, pneumonia *etc*). The prevention and/or treatment of bacterial meningitis is thus preferred.

Vaccines according to the invention may either be prophylactic (*i*.*e*. to prevent infection) or therapeutic (*i*.*e*. to treat disease after infection), but will typically be prophylactic.

### Further components of the composition

The composition of the invention will typically, in addition to the components mentioned above, comprise one or more 'pharmaceutically acceptable carriers', which include any carrier that does not itself induce the production of antibodies harmful to the individual receiving the composition. Suitable carriers are typically large, slowly metabolised macromolecules such as proteins, polysaccharides, polylactic acids, polyglycolic acids, polymeric amino acids, amino acid copolymers, trehalose (WO00/56365) and lipid aggregates (such as oil droplets or liposomes). Such carriers are well known to those of ordinary skill in the art. The vaccines may also contain diluents, such as water, saline, glycerol, *etc.* Additionally, auxiliary substances, such as wetting or emulsifying agents, pH buffering substances, and the like, may be present. A thorough discussion of pharmaceutically acceptable excipients is available in *Remington's Pharmaceutical Sciences* [*e*.*g*. ref. 88].

Immunogenic compositions used as vaccines comprise an immunologically effective amount of antigen, as well as any other of the above-mentioned components, as needed. By 'immunologically effective amount', it is meant that the administration of that amount to an individual, either in a single dose or as part of a series, is effective for treatment or prevention. This amount varies depending upon the health and physical condition of the individual to be treated, age, the taxonomic group of individual to be treated (*e*.*g*. non-human primate, primate, *etc*.), the capacity of the individual's immune system to synthesise antibodies, the degree of protection desired, the formulation of the vaccine, the treating doctor's assessment of the medical situation, and other relevant factors. It is expected that the amount will fall in a relatively broad range that can be determined through routine trials. Dosage treatment may be a single dose schedule or a multiple dose schedule (*e*.*g*. including booster doses). The vaccine may be administered in conjunction with other immunoregulatory agents.

The vaccine may be administered in conjunction with other immunoregulatory agents.

The vaccine may include an adjuvant in addition to the aluminium salt. Preferred adjuvants to enhance effectiveness of the composition include, but are not limited to: (1) oil-in-water emulsion formulations (with or without other specific immunostimulating agents such as muramyl peptides (see below) or bacterial cell wall components), such as for example (a) MF59™ (WO90/14837; Chapter 10 in ref. 1), containing 5% Squalene, 0.5% Tween 80, and 0.5% Span 85 (optionally containing MTP-PE) formulated into submicron particles using a microfluidizer, (b) SAF, containing 10% Squalane, 0.4% Tween 80, 5% pluronic-blocked polymer L121, and thr-MDP either microfluidized into a submicron emulsion or vortexed to generate a larger particle size emulsion, and (c) Ribi^{™} adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox^{™}); (2) saponin adjuvants, such as QS21 or Stimulon^{™} (Cambridge Bioscience, Worcester, MA) may be used or particles generated therefrom such as ISCOMs (immunostimulating complexes), which ISCOMS may be devoid of additional detergent *e*.*g*. WO00/07621; (3) Complete Freund's Adjuvant (CFA) and Incomplete Freund's Adjuvant (IFA); (4) cytokines, such as interleukins (*e*.*g*. IL-1, IL-2, IL-4, IL-5, IL-6, IL-7, IL-12 (WO99/44636), *etc.),* interferons (*e*.*g*. gamma interferon), macrophage colony stimulating factor (M-CSF), tumor necrosis factor (TNF), *etc*.; (5) monophosphoryl lipid A (MPL) or 3-O-deacylated MPL (3dMPL) *e*.*g*. GB-2220221, EP-A-0689454; (6) combinations of 3dMPL with, for example, QS21 and/or oil-in-water emulsions *e*.*g*. EP-A-0835318, EP-A-0735898, EP-A-0761231; (7) oligonucleotides comprising CpG motifs [Krieg Vaccine 2000, 19, 618-622; Krieg Curr opin Mol Ther 2001 3:15-24; Roman et al., Nat. Med., 1997, 3, 849-854; Weiner et al., PNAS USA, 1997, 94, 10833-10837; Davis et al., J. Immunol., 1998, 160, 870-876; Chu et al., J. Exp. Med., 1997, 186, 1623-1631; Lipford et al., Eur. J. Immunol., 1997, 27, 2340-2344; Moldoveanu et al., Vaccine, 1988,16, 1216-1224, Krieg et al., Nature, 1995, 374, 546-549; Klinman et al., PNAS USA, 1996, 93, 2879-2883; Ballas et al., J. Immunol., 1996, 157, 1840-1845; Cowdery et al., J. Immunol., 1996, 156, 4570-4575; Halpern et al., Cell. Immunol., 1996, 167, 72-78; Yamamoto et al., Jpn. J. Cancer Res., 1988, 79, 866-873; Stacey et al., J. Immunol., 1996, 157, 2116-2122; Messina et al., J. Immuno/., 1991, 147, 1759-1764; Yi et al., J. Immunol., 1996, 157, 4918-4925; Yi et al., J. Immuno/., 1996, 157, 5394-5402; Yi et al., J. Immunol., 1998, 160, 47S5-4761; and Yi et al., J. Immunol., 1998, 160, 5898-5906; International patent applications WO96/02555, WO98/16247, WO98/18810, WO98/40100, WO98/55495, WO98/37919 and WO98/52581] *i*.*e*. containing at least one CG dinucleotide, with 5-methylcytosine optionally being used in place of cytosine; (8) a polyoxyethylene ether or a polyoxyethylene ester *e*.*g*. WO99/52549; (9) a polyoxyethylene sorbitan ester surfactant in combination with an octoxynol (*e*.*g*. WO01/21207) or a polyoxyethylene alkyl ether or ester surfactant in combination with at least one additional non-ionic surfactant such as an octoxynol (*e*.*g*. WO01/21152); (10) an immunostimulatory oligonucleotide (*e*.*g.* a CpG oligonucleotide) and a saponin *e*.*g*. WO00/62800; (11) an immunostimulant and a particle of metal salt *e*.*g*. WO00/23105; (12) a saponin and an oil-in-water emulsion *e*.*g*. WO99/11241; (13) a saponin (*e*.*g*. QS21) + 3dMPL + IL-12 (optionally + a sterol) *e*.*g*. WO98/57659; (14) chitosan; (15) cholera toxin or *E.coli* heat labile toxin, or detoxified mutants thereof [89]; (16) microparticles of poly(α-hydroxy)acids, such as PLG; (17) other substances that act as immunostimulating agents to enhance the efficacy of the composition.

Muramyl peptides include N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-*sn*-glycero-3-hydroxyphosphoryloxy)-ethylamine MTP-PE), *etc.*

Once formulated, the compositions of the invention can be administered directly to the subject. The subjects to be treated can be animals; in particular, human subjects can be treated. The vaccines are particularly useful for vaccinating children and teenagers.

Typically, the immunogenic compositions are prepared as injectables, either as liquid solutions or suspensions; solid forms suitable for solution in, or suspension in, liquid vehicles prior to injection may also be prepared. The preparation also may be emulsified or encapsulated in liposomes for enhanced adjuvant effect. Direct delivery of the compositions will generally be parenteral (*e*.*g*. by injection, either subcutaneously, intraperitoneally, intravenously or intramuscularly or delivered to the interstitial space of a tissue). The compositions can also be administered into a lesion. Other modes of administration include oral and pulmonary administration, suppositories, and transdermal or transcutaneous applications (*e*.*g*. see WO98/20734), needles, and hyposprays. Dosage treatment may be a single dose schedule or a multiple dose schedule (*e*.*g*. including booster doses).

### The step of admixing antigen, aluminium salt and histidine

To make compositions of the invention, antigen, aluminium salt and histidine must be combined. It is preferred that, when the antigen and aluminium salt are mixed, the histidine should be present.

Histidine is thus present during adsorption to the aluminium salt. This compares with adding histidine to an antigen/aluminium salt combination which already exists *i*.*e*. the histidine in the process is not simply added as a buffer after antigen and aluminium salt have interacted, but instead it is present during their interaction.

In the process of the invention, therefore, antigen is preferably admixed with a histidine/aluminium salt mixture. The process of the invention may therefore comprise the following steps: (a) preparing a mixture of the aluminium salt and the histidine; and (b) admixing the antigen with said mixture. The mixture of (a) is preferably aqueous and may be prepared in aqueous conditions or may be a dried mixture which is re-hydrated prior to use.

Once one or more antigens has been adsorbed to an aluminium salt in the presence of histidine, the mixture may be combined with other antigens *e*.*g*. combined with existing diphtheria, tetanus, pertussis, polio or hepatitis B virus compositions.

### Definitions

The term "comprising" means "including" as well as "consisting" *e*.*g*. a composition "comprising" X may consist exclusively of X or may include something additional *e*.*g*. X + Y.

The term "about" in relation to a numerical value *x* means, for example, *x*±10%.

### BRIEF DESCRIPTION OF DRAWINGS

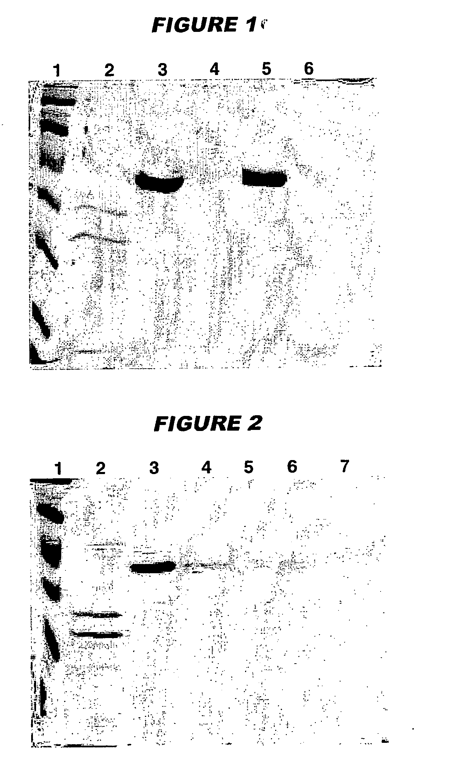

Figure 1 shows SDS-PAGE analysis of antigenic compositions following centrifugation. Lane 1 includes MW markers (220, 97, 66, 46, 30, 21, 14 kDa). OMV antigen (2µg) was used in lane 2; ΔG287 antigen was used in lanes 3 (10µg) and 4 (0.5µg). The antigen used in lanes 5 and 6 was a combination of OMV (50µg/ml) and ΔG287 (100µg/ml) with 1mg/ml aluminium oxyhydroxide; the lane 5 composition included 10mM sodium phosphate (PBS), whereas the lane 6 composition included 5mM histidine in saline solution.
Figure 2 also shows SDS-PAGE analysis of antigenic compositions following centrifugation. Lane 1 includes the same MW markers as Figure 1. OMV antigen (2.5µg) was used in lane 2; ΔG287 antigen was used in lanes 3 (2µg) and 4 (0.5µg). The antigen used in lanes 5, 6 and 7 was a combination of OMV (50µg/ml) and ΔG287 (100µg/ml) with 1mg/ml aluminium oxyhydroxide in saline solution (pH 6.5); the lane 5 composition included 2.5mM sodium phosphate, the lane 6 composition included 5mM histidine, and the lane 7 composition included 10mM histidine.

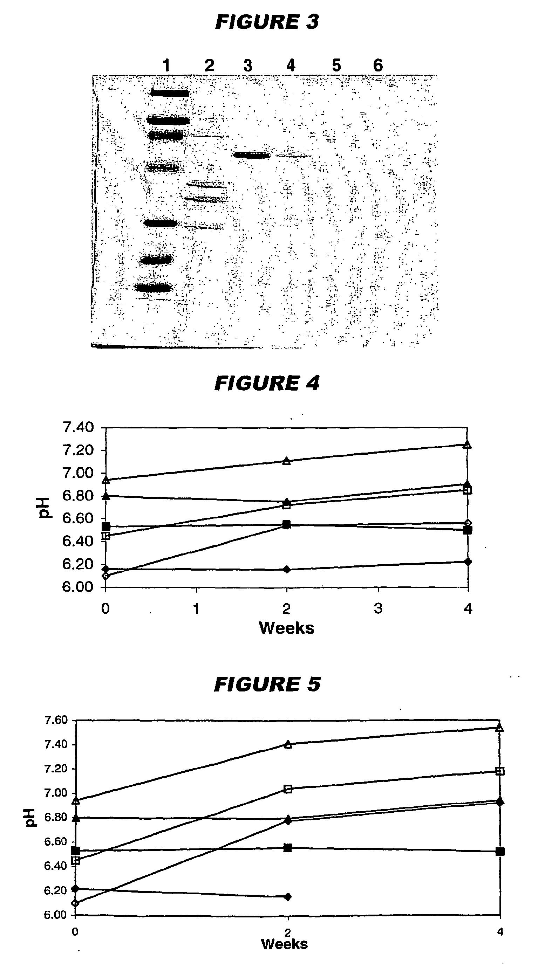

Figure 3 also shows SDS-PAGE analysis of antigenic compositions following centrifugation. Lane 1 includes the same MW markers as Figure 1. OMV antigen (2µg) was used in lane 2; ΔG287 antigen was used in lanes 3 (2µg) and 4 (0.5µg). The antigen used in lanes 5 and 6 was a combination of OMV (50µg/ml) and ΔG287 (100µg/ml) with 3.3mg/ml aluminium oxyhydroxide in saline solution (pH 6.5); the lane 5 composition included 2.5mM sodium phosphate (PBS), whereas the lane 6 composition included 5mM histidine in saline solution.
Figure 4 shows the pH stability of vaccine formulations at 4°C. Filled symbols represent vaccines buffered with 5mM histidine; open symbols represent vaccines buffered with 2.5mM sodium phosphate. The initial pH was 6.0 (diamond), 6.5 (square) or 7.0 (triangle). Figure 5 shows the same at 37°C.

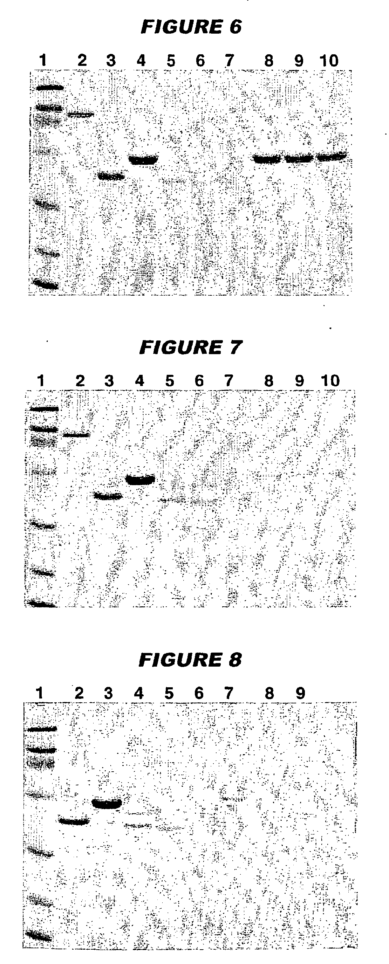

Figure 6 shows a SDS-PAGE gel for various antigens. Lane 1 contains MW markers. Lanes 2 to 6 contain markers: (2) ΔG287-953; (3) 961c; (4) 936-741; (5) New Zealand OMVs; and (6) Norwegian OMVs. Lanes 7 to 10 show supernatants of centrifuged histidine formulations of the invention after 1 month storage at 2-8°C: (7) ΔG287-953; (8) 961c + 936-741 + ΔG287-953; (9) 961c + 936-741 + ΔG287-953 + OMV_{NZ}; (10) 961c + 936-741 + ΔG287-953 + OMV_{Norway}.
Figure 7 shows the same as Figure 6, but lanes 7-10 are after storage at 36-38°C.
Figure 8 shows a SDS-PAGE gel for various antigens. Lane 1 contains MW markers. Lanes 2 to 5 contain markers: (2) 961c; (3) 936-741; (4) New Zealand OMVs; and (5) Norwegian OMVs. Lanes 6 to 9 show supernatants of centrifuged histidine formulations of the invention after 1 month storage at 2-8°C: (6) 961c; (7) 936-741; (8) OMV_{NZ}; (9) OMV_{Norway}.

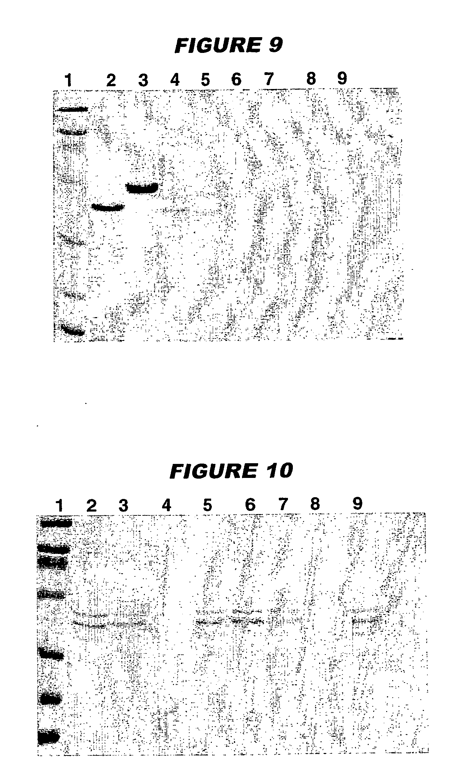

Figure 9 shows the same as Figure 8, but lanes 6-9 are after storage at 36-38°C.
Figure 10 shows a SDS-PAGE gel for New Zealand OMVs. Lane 1 contains MW markers. Lanes 2, 3, 6 & 7 contain OMV markers stored at either 2-8°C (lanes 2 & 3) or 36-38°C (lanes 6 & 7), present at either 2µg (lanes 2 & 6) or 1µg (lanes 3 & 7). Lanes 4, 5, 8 & 9 show OMVs in histidine formulations of the invention after 30 days storage at either 2-8°C (lanes 4 & 5) or 36-38°C (lanes 8 & 9). Lanes 4 & 8 show supernatant of centrifuged OMVs, whereas lanes 5 & 9 show pellets.

### MODES FOR CARRYING OUT THE INVENTION

### Example 1- pH stability and adsorption of meningococcal B '287' antigen

Reference 11 discloses a protein antigen named '287' from *N.meningitidis* serogroup B. Reference 90 discloses a form of this antigen ('ΔG287') which is truncated to remove the N-terminal amino acids up to and including its hexaglycine region. 287 and ΔG287 are both able to elicit a protective immune response in mice. References 16 to 19 disclose OMV antigens from *N.meningitidis* serogroup B. These OMVs are also able to elicit a protective immune response in mice.

These two antigens were formulated by adsorption to aluminium oxyhydroxide adjuvant. Two adjuvant concentrations (1 mg/ml and 3.3 mg/ml) were tested.

Immunisation studies in mice showed that vaccine immunogenicity is linked to the level of adsorption of the antigens to the adjuvant. To assess adsorption levels, samples of the final formulations were centrifuged at 1300 rpm for 10 minutes and the supernatant was analysed by SDS-PAGE in order to detect the presence of non-adsorbed antigen. The relevant protein standards at an appropriate concentration were loaded adjacent for quantitative comparison.

In order to maintain a stable physiological pH at 4°C and 37°C over a period of 4 weeks using sodium phosphate buffer it was found that the composition requires 10mM sodium phosphate. At this level, however, adsorption of ΔG287 was only 50% (Figure 1, lane 5). 100% adsorption could be maintained at 2.5mM sodium phosphate (Lanes 5 of Figures 2 & 3), but this composition does not have a stable pH at either 4°C or 37°C.

It was therefore necessary to find an alternative buffer system which would maintain pH stability without decreasing adsorption.

Adsorption was 95-100% using 5mM histidine (Lanes 6 of Figures 1, 2 & 3) and also using 10mM histidine (Figure 2, lane 7). In terms of adsorption, therefore, 5mM or 10mM histidine was equivalent to 2.5mM sodium phosphate in the presence of either lmg/ml (Figures 1 & 2) or 3.3mg/ml (Figure 3) aluminium oxyhydroxide.

In order to define the pH range in which the vaccine compositions are stable, three starting pH values were chosen (pH 6.0, 6.5 and 7.0) and pH stability was monitored over four weeks in the presence of either 2.5mM sodium phosphate or 5mM histidine. Stability was monitored at both 4°C and 37°C.

The antigen in all vaccines was a combination of ΔG287 (100µg/ml) and OMV (50µg/ml) adjuvanted with 3.3mg/ml aluminium oxyhydroxide.

Figure 4 shows pH stability at 4°C and Figure 5 shows pH stability at 37°C [NB - due to bacterial contamination, no measurement of the pH 6.0 histidine-buffered vaccine was possible at 4 weeks].

At both temperatures the pH tended to increase over time with 2.5mM sodium phosphate buffer but was stable in the presence of 5mM histidine buffer.

In comparison with sodium phosphate buffer, therefore, the use of histidine offers pH stability over time without reducing adsorption.

### Example 2 - adsorption of meningococcal C saccharide antigen

Saccharide conjugates tend to degrade by hydrolysis [7,8] when present in solution ('liquid' vaccines). Conjugates can be lyophilised to avoid this [7], but this requires adjuvant to be added at the point of reconstitution. It would be preferable to have a liquid form of the vaccine in which the saccharide is not subject to hydrolytic degradation.

This was investigated for a conjugate of meningococcus serogroup C oligosaccharide on CRM₁₉₇ carrier protein [20]. CRM₁₉₇ is acidic and thus does not completely adsorb to negatively charged aluminium phosphates. Histidine, however, is positively charged and it was thought that this might be able to mask the negative charge. Histidine buffer was thus tested with the aim of improving adsorption of MenC-CRM₁₉₇ to aluminium hydroxyphosphate.

Antigen adsorption was evaluated in the presence and absence of histidine buffer by measuring protein concentration in the vaccine supernatant using the BCA protein assay, after centrifugation to separate the adjuvant pellet. The vaccines were formulated as 20µg/ml oligosaccharide and 45µg/ml CRM₁₉₇ protein. Results were as follows:

| **Antigen** | **Adjuvant** | **[Histidine] (mM)** | **Protein (**µ**g**/**ml)** |
|---|---|---|---|
| MenC-CRM₁₉₇ | Hydroxyphosphate Al³⁺=0.6mg/ml | 0 | 42.4 |
| | | 5 | 28.6 |
| | | 10 | 21.7 |

Antigen adsorption thus improves when histidine is present in the formulation: adsorption is about 6% in the absence of histidine; 5mM histidine increases this to 36%; 10mM histidine increases adsorption to almost 52%.

Histidine is thus a useful additive for improving the adsorption of antigens to aluminium hydroxyphosphate.

### Example 3 - adsorption of meningococcal B NadA antigen

NadA (Neisserial adhesin A) from serogroup B *N.meningitidis* is disclosed as protein '961' in ref. 11 (SEQ IDs 2943 & 2944) and as 'NMB1994' in ref.13 (see also GenBank accession numbers 11352904 & 7227256). Allelic forms of NadA are disclosed in reference 91. Preferred forms of NadA lack the C-terminus anchor domain ('961c').

961c (100µg/ml) was adsorbed onto aluminium oxyhydroxide (3mg/ml) in the presence of 10mM histidine buffer, pH 6.5. After 4 weeks of storage at either 2-8°C or at 36-38°C, the antigen remained 100% adsorbed (Figures 8 & 9, lane 6). The pH of the composition was 6.44 at time zero and after 4 weeks of storage rose very slightly to 6.48 (2-8°C) or 6.47 (36-38°C).

### Example 4 - adsorption of meningococcal B hybrid antigens

Reference 92 discloses hybrid expression of meningococcal B antigens. One such hybrid is 'ΔG287_{nz}-953' and another is '936-741'. These two hybrids (100µg/ml) were each adsorbed onto aluminium oxyhydroxide (3mg/ml) in the presence of 10mM histidine buffer, pH 6.3. After 4 weeks of storage at either 2-8°C or at 36-38°C, 'ΔG287_{nz}-953' remained 100% adsorbed (Figures 6 & 7, lane 7), with pH rising slightly from 6.44 to 6.52 (2-8°C) or 6.53 (36-38°C). '936-741' remained 100% adsorbed at 36-38°C (Figure 9, lane 7) but was ∼99% adsorbed at 2-8°C (Figure 8, lane 7), with pH rising slightly from 6.33 to 6.37 (2-8°C) or 6.38 (36-38°C).

### Example 5 - adsorption of meningococcal OMVs

As mentioned above, OMV vaccines from meningococcus B are well known. OMVs were prepared from the Norwegian strain of meningococcus B or from a New Zealand strain (394/98). These two OMV preparations (50µg/ml) were adsorbed onto aluminium oxyhydroxide (3mg/ml) in the presence of 10mM histidine buffer, pH 6.5. After 4 weeks of storage at either 2-8°C or at 36-38°C, both OMV preparations remained 100% adsorbed (Figures 8 & 9, lanes 8 & 9). For the Norwegian OMVs, pH rose slightly from 6.39 to 6.42 over 4 weeks at both storage temperatures. For the New Zealand OMVs, pH rose slightly from 6.40 to 6.42 (2-8°C) or 6.43 (36-38°C).

New Zealand OMVs were alternatively formulated with 5mM histidine. Starting with pure water, the aluminium oxyhydroxide was added, followed by histidine, with 10 minutes mixing. The OMVs were then added and mixed for 15 minutes. NaCl was then added followed by 10 minutes further mixing. The final composition was 3.3mg/ml aluminium oxyhydroxide, 7.5mM NaCl, 5mM histidine, 100µg/ml OMV, pH 6.42.

During storage at either 2-8°C or 36-36°C, pH and OMV adsorption varied as follows:

| | **pH** | | **% Adsorption** | |
|---|---|---|---|---|
| | 2-8°C | 36-38°C | 2-8°C | 36-38°C |
| **Time zero** | 6,42 | 6.42 | 100 | 100 |
| **15 days** | 6,36 | 6,37 | 100 | 100 |
| **30 days** | 6,35 | 6,34 | 100 | 100 |

A comparison of lanes 4 & 5 (2-8°C) or lanes 8 & 9 (36-38°C) in Figure 10 shows that OMVs remain adsorbed after 1 month of storage.

### Example 6 - adsorption of mixtures of meningococcal OMVs and protein antigens

961c, ΔG287_{nz}-953 and 936-741 were mixed at 100µg/ml of each antigen and the mixture was adsorbed onto aluminium oxyhydroxide (3mg/ml) in the presence of 10mM histidine buffer, pH 6.3. In two further formulations, OMVs were included (50µg/ml) from either Norwegian or New Zealand strain meningococcus B.

All antigens in the three mixtures (Figures 6 & 7, lanes 8-10) showed 100% adsorption after 4 weeks of storage at either 2-8°C or at 36-38°C, except for 936-741 which was -96% adsorbed in all three mixtures at 2-8°C and ∼99% adsorbed at 36-38°C. The pH of each of the three mixtures rose slightly from 6.53 at time zero to 6.62 after 4 weeks at 2-8°C. At 36-38°C, the pH of three mixtures rose to 6.71±0.02.

The individual antigens brought residual phosphate ions into the mixture from their own PBS. Phosphate ions were sometimes present at between 3 and 5 mM in the combined antigen mixture. In the presence of these high concentrations of residual phosphate buffer, it was difficult to stabilise pH within 6.0 to 7.0, even with 5mM histidine. When histidine was increased to 10mM, however, pH was stabilised. Furthermore, the antigens remained adsorbed even after 1 month of storage at either 2-8°C or at 36-38°C.

### Example 7 - adsorption of meningococcal A saccharide antigen

Reference 94 discloses CRM₁₉₇ conjugates of capsular oligosaccharide from serogroup A meningococcus. The conjugates are not fully stable and are therefore prepared in lyophilised form, ready for re-constitution at the time of administration. The lyophilised form was prepared to have components which give the following composition after reconstitution into a unit dose:

| **Component** | **Concentration** |
|---|---|
| CRM-MenA | 20µg saccharide/ml |
| Potassium phosphate buffer | 5 mM |
| Mannitol | 15 mg/ml |

This composition has no adjuvant, so an adjuvant was prepared for its reconstitution:

| **Component** | **Concentration** |
|---|---|
| Aluminium oxyhydroxide | 0.68 mg Al³⁺/ml |
| Histidine buffer | 10 mM |
| Sodium chloride | 9 mg/ml |
| Tween 80 | 0.005% |
| PH | 7.2±0.05 |

| | |
|---|---|
| * amorphous hydroxyphosphate, PO₄/Al molar ratio between 0.84 and 0.92 | |

### Example 8 - adsorption of meningococcal C, W135 and Y saccharide antigens

Reference 94 discloses CRM₁₉₇ conjugates of capsular oligosaccharides from meningococcus serogroups C, W135 and Y . A trivalent mixture of the three conjugates either adsorbed onto an aluminium oxyhydroxide adjuvant (2mg/ml) or an aluminium hydroxyphosphate adjuvant (0.6mg/ml Al³⁺) was prepared. The compositions of the two trivalent mixtures were as follows:

| **Component** | **Concentration** | **Concentration** |
|---|---|---|
| Aluminium oxyhydroxide | 0.68 mg Al³⁺/ml | - |
| Aluminium hydroxyphosphate* | - | 0.6mg Al³/ml |
| CRM-MenC | 20µg saccharide/ml | 20µg saccharide/ml |
| CRM-MenY | 20µg saccharide/ml | 20µg saccharide/ml |
| CRM-MenW135 | 20µg saccharide/ml | 20µg saccharide/ml |
| Sodium phosphate buffer | - | 10mM |
| Histidine buffer | 10 mM | - |
| Sodium chloride | 9 mg/ml | 9mg/ml |
| Tween 80 | 0.005% | 0.005% |

| | | |
|---|---|---|
| * amorphous hydroxyphosphate, PO₄/Al molar ratio between 0.84 and 0.92 | | |

For the oxyhydroxide/histidine formulation, stability of the saccharide components either in the bulk mixture or after packaging into vials was as follows:

| **Time (days)** | **Stored at 2-8°C** | | **Stored at 36-38°C** | |
|---|---|---|---|---|
| | **Free saccharide (µg/ml)** | **Free saccharide %** | **Free saccharide (µg/ml)** | **Free saccharide %** |
| MenC bulk | | | | |
| 0 | <1.2 | <6 | <1.2 | <6 |
| 15 | <1.2 | <6 | <1.2 | <6 |
| 30 | <1.2 | <6 | <1.2 | <6 |

| MenC vials | | | | |
|---|---|---|---|---|
| 0 | <1.2 | <6 | <1.2 | <6 |
| 15 | <1.2 | <6 | <1.2 | <6 |
| 30 | <1.2 | <6 | 1.3 | 6.6 |

| MenW135 bulk | | | | |
|---|---|---|---|---|
| 0 | 2.5 | 12.5 | 2.5 | 12.5 |
| 15 | 2.3 | 11.4 | 3.4 | 16.8 |
| 30 | 2.3 | 11.5 | 3.5 | 17.3 |

| MenW135 vials | | | | |
|---|---|---|---|---|
| 0 | 2.1 | 10.6 | 2.1 | 10.6 |
| 15 | 2.3 | 11.7 | 2.7 | 13.3 |
| 30 | 20. | 10.2 | 3.3 | 16.3 |

| MenY bulk | | | | |
|---|---|---|---|---|
| 0 | 1.7 | 8.3 | 1.7 | 8.3 |
| 15 | <1.3 | <6.3 | 2.0 | 10.2 |
| 30 | 1.3 | 6.3 | 2.4 | 12.2 |

| MenY vials | | | | |
|---|---|---|---|---|
| 0 | 1.4 | 7.1 | 1.4 | 7.1 |
| 15 | 1.5 | 7.6 | 2.1 | 10.7 |
| 30 | 1.3 | 6.3 | 2.9 | 14.3 |

Free saccharide levels are thus stable for at least 1 month at 2-8°C, before and after packaging.

Under thermal stress conditions, small increases in free saccharide are seen over time for MenW135 and MenY, but MenC remains stable.

Over the 30 days, pH in vials and bulk was stable at 7.15±0.05 at both storage temperatures.

### Example 9 - adsorption of meningococcal A, C, W135 and Y saccharide antigens

The two trivalent liquid compositions of example 8 were diluted and 0.5ml used to reconstitute the lyophilised MenA conjugate of example 7. The resulting tetravalent mixture was administered to ten Balb/c mice (female 6-8 weeks old) per group by subcutaneous injection at day 0 and 28. The mixture contained 2µg of each saccharide conjugate per dose, which represents 1/5 of the single human dose (SHD). Controls were saline or unconjugated homologous polysaccharides. Bleedings were performed before immunization and then at day 42, with sera stored at -70°C.

All the conjugates used were safe and immunogenic in the animals. GMT post-II ELISA titres (with 95% confidence intervals) were as follows:

| **Vaccine** | **Adjuvant** | **A** | **Y** | **W135** | **C** |
|---|---|---|---|---|---|
| MenA (lyophilised and resuspended) | Hydroxyphosphate | 172 (69-439) | - | - | - |
| | Oxyhydroxide | 619 (419-906) | - | - | - |
| MenY | Hydroxyphosphate | | 328 (147-731) | - | - |
| | Oxyhydroxide | - | 452 (344-593) | - | - |
| MenW | Hydroxyphosphate | - | - | 80 (28-225) | - |
| | Oxyhydroxide | - | - | 277 (185-411) | - |
| MenC | Hydroxyphosphate | - | - | - | 317 (152-659) |
| | Oxyhydroxide | - | - | - | 723 (615-851) |
| MenA (lyophilized) + MenC,W135,Y | Hydroxyphosphate | 32 (15-68) | 397 (252-627) | 99 (35-288) | 114 (53-246) |
| | Oxyhydroxide | 206 (112-372) | 141 (97-205) | 139 (76-251) | 163 (122-218) |

Typically, therefore, titres are higher in the aluminium oxyhydroxide + histidine groups. Serum bactericidal titres were also generally better in the aluminium oxyhydroxide + histidine groups.

In parallel experiments, mice were immunised as described above but the vaccine compositions contained different ratios of the various oligosaccharide conjugates. Lyophilised MenA oligo-conjugate was used in all experiments. ELISA titres were as follows:

| **Antigen quantity (**µ**g/dose)** | | | | **Aluminium adjuvant** | **GMT ELISA (95% confidence interval)** | | | |
|---|---|---|---|---|---|---|---|---|
| **A** | **C** | **W135** | **Y** | | **A** | **C** | **W135** | **Y** |
| 4 | 2 | 2 | 2 | Hydroxyphosphate | 177 | 367 | 239 | 239 |
| | | | | | (107-291) | (263-510) | (135-424) | (184-311) |
| 4 | 2 | 2 | 2 | Oxyhydroxide | 390 | 494 | 338 | 158 |
| | | | | | (313-486) | (345-706) | (266-430) | (96-260) |
| 2 | 2 | 2 | 2 | Hydroxyphosphate | 132 | 582 | 143 | 247 |
| | | | | | (59-296) | (268-1155) | (75-272) | (152-400) |
| 2 | 2 | 2 | 2 | Oxyhydroxide | 337 | 569 | 171 | 100 |
| | | | | | (239-476) | (462-679) | (117-251) | (59-169) |

A second set of experiments was performed using a dosage of 2 µg/ml saccharide for MenA and MenC, half that dosage for MenY, and a quarter dosage for MenW135. ELISA titres were as follows:

| **Antigen quantity (µg/dose)** | | | | **Aluminium adjuvant** | **GMT ELISA (95% confidence interval)** | | | |
|---|---|---|---|---|---|---|---|---|
| **A** | **C** | **W135** | **Y** | | **A** | **C** | **W135** | **Y** |
| 2 | 2 | 2 | 2 | Hydroxyphosphate | 32 | 114 | 99 | 397 |
| | | | | | (15-68) | (53-246) | (35-288) | (252-627) |
| | | | | Oxyhydroxide | 206 | 163 | 139 | 141 |
| | | | | | (112-372) | (122-218) | (76-251) | (97-205) |
| 2 | 2 | 1 | 0.5 | Hydroxyphosphate | 96 | 238 | 42 | 315 |
| | | | | | (49-187) | (101-561) | (20-89) | (114-867) |
| | | | | Oxyhydroxide | 293 | 267 | 83 | 244 |
| | | | | | (144-597) | (158-451) | (43-163) | (152-392) |

At least for serogroups A, C and W135, therefore, the oxyhydroxide + histidine formulation generally gives better titres than hydroxyphosphate at these different antigen ratios.

It will be understood that the invention has been described by way of example only.

### REFERENCES

*1-* Vaccine Design: subunit & adjuvant approach (1995) Powell & Newman (ISBN: 030644867X)
2 - International patent application WO93/24148.
3 - International patent application WO97/00697.
4 - International patent application WO99/48525.
5 - Burrell et al. (2000) Vaccine 18:2188-2192.
6 - Burrell et a/. (1999) Vaccine 17:2599-2603.
7 - Corbel (1996) Dev Biol Stand 87:113-124.
8 - Sturgess et al. (1999) Vaccine 17:1169-1178.
9 - International patent application WO99/24578.
10 - International patent application WO99/36544.
11 - International patent application WO99/57280.
12 - International patent application WO00/22430.
13 - Tettelin et al. (2000) Science 287:1809-1815.
14 - International patent application WO96/29412.
15 - Pizza et al. (2000) Science 287:1816-1820.
16 - International patent application WO01/52885.
17 - Bjune et al. (1991) Lancet 338(8775):1093-1096.
18 - Fukasawa et al. (1999) Vaccine 17:2951-2958.
19 - Rosenqvist et al. (1998) Dev. Biol. Stand. 92:323-333.
20 - Costantino et al. (1992) Vaccine 10:691-698.
21 - Costantino et al. (1999) Vaccine 17:1251-1263.
22 - Watson (2000) Pediatr Infect Dis J 19:331-332.
23 - Rubin (2000) Pediatr Clin North Am 47:269-285, v.
24 -Jedrzejas (2001) Microbiol Mol Biol Rev 65:187-207.
25 - Gustafsson et al. (1996) N. Engl. J. Med. 334:349-355.
26 - Rappuoli et al. (1991) TIBTECH 9:232-238.
27 - Vaccines (1988) eds. Plotkin & Mortimer. ISBN 0-7216-1946-0.
28 - Del Guidice et al. (1998) Molecular Aspects of Medicine 19:1-70.
29 - International patent application WO93/18150.
30 - International patent application WO99/53310.
31 - International patent application WO98/04702.
32 - International patent application WO02/02606.
33 - Kalman et al. (1999) Nature Genetics 21:385-389.
34 - Read et al. (2000) Nucleic Acids Res 28:1397-406.
35 - Shirai et al. (2000) J. Infect. Dis. 181 (Suppl 3):S524-S527.
36 - International patent application WO99/27105.
37 - International patent application WO00/27994.
38 - International patent application WO00/37494.
39 - International patent application WO99/28475.
40 - Ross et al. (2001) Vaccine 19:4135-4142.
41 - McMichael (2000) Vaccine 19 Suppl 1:S101-107.
42 - Schuchat (1999) Lancet 353(9146):51-6.
43 - International patent application WO02/34771.
44 - Dale (1999) Infect Dis Clin North Am 13:227-43, viii.
45 - Ferretti et al. (2001) PNAS USA 98: 4658-4663.
46 - Kuroda et al. (2001) Lancet 357(9264):1225-1240; see also pages 1218-1219.
47 - J Toxicol Clin Toxicol (2001) 39:85-100.
48 - Demicheli et al. (1998) Vaccine 16:880-884.
49 - Stepanov et al. (1996) J Biotechnol 44:155-160.
50 - Bell (2000) Pediatr Infect Dis J 19:1187-1188.
51 - Iwarson (1995) APMIS 103:321-326.
52 - Gerlich et al. (1990) Vaccine 8 Suppl:S63-68 & 79-80.
53 - Hsu et al. (1999) Clin Liver Dis 3:901-915.
54 - Sutter et al. (2000) Pediatr Clin North Am 47:287-308.
55 - Zimmerman & Spann (1999) Am Fam Physician 59:113-118, 125-126.
56 - Dreesen (1997) Vaccine 15 Suppl:S2-6.
57 - MMWR Morb Mortal Wkly Rep 1998 Jan 16;47(1):12, 19.
58 - Ingram (2001) Trends Neurosci 24:305-307.
59 - Rosenberg (2001) Nature 411:380-384.
60 - Moingeon (2001) Vaccine 19:1305-1326.
61 - Ramsay et al. (2001) Lancet 357(9251):195-196.
62 - Lindberg (1999) Vaccine 17 Suppl 2:S28-36.
63 - Buttery & Moxon (2000) J R Coll Physicians Lond 34:163-168.
64 - Ahmad & Chapnick (1999) Infect Dis Clin North Am 13:113-133, vii.
65 - Goldblatt (1998) J. Med. Microbiol. 47:563-567.
66 - European patent 0 477 508.
67 - US patent 5,306,492.
68 - International patent application WO98/42721.
69 - Conjugate Vaccines (eds. Cruse et al.) ISBN 3805549326, particularly vol. 10:48-114.
70 - Hermanson (1996) Bioconjugate Techniques ISBN: 0123423368 or 012342335X.
71 - European patent application 0372501.
72 - European patent application 0378881.
73 - European patent application 0427347.
74 - International patent application WO93/17712.
75 - International patent application WO98/58668.
76 - European patent application 0471177.
77 - International patent application WO00/56360.
78 - International patent application WO00/61761.
79 - Robinson & Torres (1997) Seminars in Immunology 9:271-283.
80 - Donnelly et al. (1997) Annu Rev Immunol 15:617-648.
81 - Scott-Taylor & Dalgleish (2000) Expert Opin Investig Drugs 9:471-480.
82 - Apostolopoulos & Plebanski (2000) Curr Opin Mol Ther 2:441-447.
83 - Ilan (1999) Curr Opin Mol Ther 1:116-120.
84 - Dubensky et al. (2000) Mol Med 6:723-732.
85 - Robinson & Pertmer (2000) Adv Virus Res 55:1-74.
86 - Donnelly et al. (2000) Am J Respir Crit Care Med 162(4 Pt 2):S 190-193.
87 - Davis (1999) Mt Sinai J Med 66:84-90.
88 - Gennaro (2000) Remington: The Science and Practice of Pharmacy. 20th edition, ISBN: 0683306472.
89 - WO93/13202.
90 - International patent application WO01/64922.
91 - International patent application WO03/010194.
92 - International patent application WO01/64920.
94 - International patent application WO03/007985.
95 - International patent application WO00/57906.

## Claims

1. A process for producing an immunogenic composition comprising a mixture of one or more antigens, an aluminium salt and histidine wherein the process comprises: a first step of admixing (i) the aluminium salt and (ii) histidine, to give a histidine/aluminium salt admixture; and a second step of admixing (i) said histidine/aluminium salt admixture and (ii) one or more antigens, wherein one or more antigen(s) is/are adsorbed to the aluminium salt and wherein the antigen is a bacterial antigen selected from the group consisting of:
- a protein antigen from *N.meningitidis*;
- an outer-membrane vesicle (OMV) preparation from *N.meningitidis*;
- a saccharide antigen from *N.meningitidis.*

2. The process of claim 1, wherein the antigen is from *Neisseria meningitidis* serogroup B.

3. The process of claim 1, wherein the saccharide antigen is a conjugated saccharide antigen.

4. The process of any preceding claim, wherein the aluminium salt is a hydroxide or a phosphate, or a mixture of two or more of said salts.

5. The process of any preceding claim, wherein aluminium salt is aluminium hydroxyphosphate and the antigen is an acidic antigen.

6. The process of any preceding claim, wherein the concentration of histidine in the composition is between 1mM and 100mM.

7. The process of any preceding claim, wherein the concentration of histidine in the composition is between about 5mM and about 10mM.

8. The process of any preceding claim, wherein the composition further comprises a sodium salt.

9. The process of claim 8, wherein the concentration of the sodium salt is between about 2.5mM and about 5mM.

10. The process of any preceding claim, wherein the pH of the composition is between 6 and 7.

11. The process of any preceding claim, wherein the composition further comprises a pharmaceutically acceptable carrier.

12. The process of any preceding claim, wherein the composition further comprises more than one antigen.

13. The process of claim 12, wherein more than one of the antigens is adsorbed to an aluminium salt.

14. The process of claim 12 or claim 13, comprising 2, 3, 4, 5, 6 or 7 of the following: an antigen from *Bordetella pertussis*; a diphtheria antigen; a tetanus antigen; an antigen from hepatitis B virus; a saccharide antigen from *Haemophilus influenzae*; inactivated polio virus; and a saccharide antigen from *N.meningitidis* serogroup C.

15. An immunogenic composition consisting of a mixture of (i) one or more antigens selected from the group consisting of a protein antigen from *N.meningitidis;* an outer-membrane vesicle (OMV) preparation from *N.meningitidis* and a saccharide antigen from *N.meningitidis*, (ii) an aluminium salt and (iii) histidine, wherein the one or more antigens are adsorbed to the aluminium salt, obtainable by a process as claimed in any one of claims 1 to 13.

16. The composition as claimed in claim 15 for use as a medicament.

17. The composition of claim 16, wherein the medicament is a vaccine.

18. The use of the composition of claim 15 in the manufacture of a medicament for raising an immune response in a mammal against the antigen(s).

19. The use of claim 18, wherein the medicament is a vaccine.

20. The use of claim 18, wherein the mammal is a human.

## Patentansprüche

1. Verfahren zur Herstellung einer immunogenen Zusammensetzung, die eine Mischung aus einem oder mehreren Antigenen, einem Aluminiumsalz und Histidin umfasst, bei dem man in einem ersten Schritt (i) das Aluminiumsalz und (ii) Histidin vermischt, um eine Histidin/Aluminiumsalz-Mischung zu erhalten; und bei man in einem zweiten Schritt (i) die Histidin/Aluminiunsalz-Mischung und (ii) ein oder mehrere Antigene vermischt, wobei ein oder mehrere Antigen(e) an das Aluminiumsalz absorbiert ist/sind, und wobei das Antigen ein bakterielles Antigen ist, das ausgewählt ist aus der Gruppe bestehend aus:
- einem Protein-Antigen von *N. meningitidis;*
- einer Zubereitung von Vesikeln der äußeren Membran (outer membrane vesicle; OMV) von *N. meningitidis*
- einem Saccharid-Antigen von *N. meningitidis.*

2. Verfahren nach Anspruch 1, wobei das Antigen von *Neisseria meningitidis* Serogruppe B stammt.

3. Verfahren nach Anspruch 1, wobei das Saccharid-Antigen ein konjugiertes Saccharid-Antigen ist.

4. Verfahren nach einem der vorhergehenden Ansprüche, wobei das Aluminiumsalz ein Hydroxid oder ein Phosphat oder eine Mischung aus zwei oder mehreren dieser Salze ist.

5. Verfahren nach einem der vorhergehenden Ansprüche, wobei das Aluminiumsalz Aluminium-Hydroxyphosphat ist und das Antigen ein saures Antigen ist.

6. Verfahren nach einem der vorhergehenden Ansprüche, wobei die Konzentration von Histidin in der Zusammensetzung zwischen 1 mM und 100 mM ist.

7. Verfahren nach einem der vorhergehenden Ansprüche, wobei die Konzentration von Histidin in der Zusammensetzung zwischen 5 mM und 10 mM ist.

8. Verfahren nach einem der vorhergehenden Ansprüche, wobei die Zusammensetzung ferner ein Natriumsalz umfasst.

9. Verfahren nach Anspruch 8, wobei die Konzentration des Natriumsalzes zwischen etwa 2,5 mM und etwa 5 mM liegt.

10. Verfahren nach einem der vorhergehenden Ansprüche, wobei der pH-Wert der Zusammensetzung zwischen 6 und 7 ist.

11. Verfahren nach einem der vorhergehenden Ansprüche, wobei die Zusammensetzung ferner einen pharmazeutisch akzeptablen Träger umfasst.

12. Verfahren nach einem der vorhergehenden Ansprüche, wobei die Zusammensetzung ferner mehr als ein Antigen umfasst.

13. Verfahren nach Anspruch 12, wobei mehr als eines der Antigene an ein Aluminiumsalz adsorbiert ist.

14. Verfahren nach Anspruch 12 oder 13, das 2, 3, 4, 5, 6, oder 7 der folgenden Dinge umfasst: ein Antigen von *Bordetella pertussis;* ein Diphtherie-Antigen; ein Tetanus-Antigen; ein Antigen von Hepatitis B-Virus; ein Saccharid-Antigen von *Haemophilus influenzae;* inaktiviertes Polio-Virus; und ein Saccharid-Antigen von *N. meningitidis* Serogruppe C.

15. Immunogene Zusammensetzung, die aus einer Mischung von (i) einem oder mehreren Antigen(en), welche ausgewählt sind aus der Gruppe bestehend aus einem Protein-Antigen von *N. meningitidis;* einer Zubereitung von Vesikeln der äußeren Membran (outer membrane vesicle; OMV) von *N*. *meningitidis* und einem Saccharid-Antigen von *N*. *meningitidis;* (ii) einem Aluminiumsalz und (iii) Histidin besteht, wobei das eine Antigen oder die mehreren Antigene an das Aluminiumsalz adsorbiert sind, wobei die Zusammensetzung durch ein Verfahren nach einem der Ansprüche 1 bis 13 erhältlich ist.

16. Zusammensetzung nach Anspruch 15 zur Verwendung als Medikament.

17. Zusammensetzung nach Anspruch 16, wobei das Medikament ein Impfstoff ist.

18. Verwendung der Zusammensetzung nach Anspruch 15 bei der Herstellung eines Medikaments zur Erzeugung einer Immunantwort gegen das/die Antigen(e) in einem Säugetier.

19. Verwendung nach Anspruch 18, wobei das Medikament ein Impfstoff ist.

20. Verwendung nach Anspruch 18, wobei das Säugetier ein Mensch ist.

## Revendications

1. Procédé de production d'une composition immunogène comprenant un mélange d'un ou plusieurs antigènes, un sel d'aluminium et de l'histidine, dans lequel le procédé comprend : une première étape de mélange (i) du sel d'aluminium et (ii) de l'histidine, pour donner un mélange histidine/ sel d'aluminium ; et une seconde étape de mélange (i) dudit mélange histidine/sel d'aluminium et (ii) d'un ou plusieurs antigènes, où l'antigène ou les antigènes est/sont adsorbés sur le sel d'aluminium et l'antigène est un antigène bactérien choisi dans le groupe consistant en :
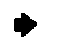 un antigène de protéine de *N. meningitidis ;*
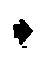 une préparation de vésicule de membrane externe (OMV) de *N. meningitidis ;*
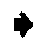 un antigène de saccharide de *N. meningitidis.*

2. Procédé selon la revendication 1, dans lequel l'antigène provient du sérogroupe B de *Neisseria meningitidis.*

3. Procédé selon la revendication 1, dans lequel l'antigène de saccharide est un antigène de saccharide conjugué.

4. Procédé selon l'une quelconque des revendications précédentes, dans lequel le sel d'aluminium est un hydroxyde ou un phosphate, ou un mélange de deux desdits sels ou plus.

5. Procédé selon l'une quelconque des revendications précédentes, dans lequel le sel d'aluminium est l'hydroxyphosphate d'aluminium et l'antigène est un antigène acide.

6. Procédé selon l'une quelconque des revendications précédentes, dans lequel la concentration en histidine dans la composition est comprise entre 1 mM et 100 mM.

7. Procédé selon l'une quelconque des revendications précédentes, dans lequel la concentration en histidine dans la composition est comprise entre environ 5 mM et environ 10 mM.

8. Procédé selon l'une quelconque des revendications précédentes, dans lequel la composition comprend en outre un sel de sodium.

9. Procédé selon la revendication 8, dans lequel la concentration en le sel de sodium est comprise entre environ 2,5 mM et environ 5 mM.

10. Procédé selon l'une quelconque des revendications précédentes, dans lequel le pH de la composition est compris entre 6 et 7.

11. Procédé selon l'une quelconque des revendications précédentes, dans lequel la composition comprend en outre un support pharmaceutiquement acceptable.

12. Procédé selon l'une quelconque des revendications précédentes, dans lequel la composition comprend en outre plus d'un antigène.

13. Procédé selon la revendication 12, dans lequel plus de l'un des antigènes est adsorbé sur un sel d'aluminium.

14. Procédé selon la revendication 12 ou la revendication 13, comprenant 2, 3, 4, 5, 6 ou 7 des éléments suivants : un antigène de *Bordetella pertussis ;* un antigène de diphtérie ; un antigène de tétanos ; un antigène du virus de l'hépatite B ; un antigène de saccharide de *Haemophilus influenza ;* le virus du polio inactivé ; et un antigène de saccharide du sérogroupe C de *N*. *meningitidis.*

15. Composition immunogène constituée d'un mélange de (i) un ou plusieurs antigènes choisis dans le groupe consistant en un antigène de protéine de *N*. *meningitidis ;* une préparation de vésicule de membrane externe (OMV) de *N*. *meningitidis* et un antigène de saccharide de *N*. *meningitidis* ; (ii) un sel d'aluminium et (iii) de l'histidine, où l'antigène ou les antigènes sont adsorbés sur le sel d'aluminium, pouvant être obtenus par un procédé tel que revendiqué dans l'une quelconque des revendications 1 à 13.

16. Composition selon la revendication 15 à utiliser comme médicament.

17. Composition selon la revendication 16, dans laquelle le médicament est un vaccin.

18. Utilisation de la composition de la revendication 15, dans la fabrication d'un médicament destiné à améliorer une réponse immunitaire chez un mammifère contre l'antigène ou les antigènes.

19. Utilisation selon la revendication 18, dans laquelle le médicament est un vaccin.

20. Utilisation selon la revendication 18, dans laquelle le mammifère est un être humain.
